# EUROPÄISCHE PATENTSCHRIFT

(11) **EP 2 727 627 B1**
(45) Veröffentlichungstag und Bekanntmachung des Hinweises auf die Patenterteilung: **20.12.2017**
(21) Anmeldenummer: 13189589.8
(22) Anmeldetag: 21.10.2013
(51) Int. Cl.: A61Q 11/00, A61K 8/67, A61K 8/42, A61K 8/44, A61K 8/46

(54) **Mund- und Zahnpflege - und -reinigungsmittel mit Tocopherylacetat**
Oral and dental care and cleaning agents with Tocopherol acetate
Produit de nettoyage et de soin de la bouche et des dents contenant de l'acétate de tocophérol

(30) Priorität: 06.11.2012 DE 102012220154
(43) Veröffentlichungstag der Anmeldung: 07.05.2014
(73) Patentinhaber: Henkel AG & Co. KGaA, 40589 Düsseldorf (DE)
(72) Erfinder: Welß, Thomas, 40591 Düsseldorf (DE); Miehlich, Kristin, 42119 Wuppertal (DE); Schmitz, Benedikt, 40589 Düsseldorf (DE); Giesen, Melanie, 47608 Geldern (DE)

(56) Entgegenhaltungen:
- WO-A1-2011/077983
- JP-A- H1 149 654
- JP-A- 2001 278 757
- JP-A- 2006 104 101
- JP-A- 2009 084 277
- JP-A- 2012 097 057
- US-A1- 2002 012 644
- US-A1- 2008 241 117

## Beschreibung

Die Anmeldung betrifft Mund- und Zahnpflege sowie Mund- und Zahnreinigungsmittel enthaltend die spezielle Kombination aus Vitamin E, anionischen und amphoteren Tensiden. Weiterhin betrifft die Anmeldung ein kosmetisches Verfahren zur Reinigung der Zähne bei gleichzeitiger Vitalisierung von Zahnfleisch und Mundschleimhaut sowie die kosmetische Verwendung von Vitamin E zur Vitalisierung des Zahnfleischs und der Mundschleimhaut.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Übliche Zahncremes enthalten Schaummittel bzw. Tenside in Mengen bis zu 5 Gew.-%. Insbesondere anionische Tenside und amphotere Tenside wie beispielsweise Betaine setzen die Oberflächenspannung herab und begünstigen die gleichmäßige Verteilung und reinigende Wirkung einer Zahncreme. Darüber hinaus sind sie entscheidend für das Mundgefühl der Zahncreme.

Tenside sind somit im Hinblick auf die Reinigung und Gesundheit der Zähne essentielle Inhaltsstoffe von zentraler Bedeutung. Während der Anwendung werden die entsprechenden Formulierungen zur Zahnreinigung bzw. Zahnpflege jedoch nicht nur auf den Zähnen appliziert, sondern kommen zwangsläufig auch mit dem Zahnfleisch bzw. der Mundschleimhaut in Kontakt. Der zu intensive Kontakt von Zahnfleisch und Mundschleimhaut mit Tensiden kann jedoch auch mit Nachteilen verbunden sein.

Der Mundraum ist mit einer befeuchteten Schleimhaut (Mucosa) ausgekleidet. Diese besteht generell aus zwei Lagen: einem mehrschichtigen, in der Regel unverhornten Plattenepithel und dem darunter liegenden Bindegewebe. Im Gegensatz zur normalen Haut besitzt die Schleimhaut keine Hornschicht. Als Zahnfleisch wird der epitheliale Bestandteil des Zahnhalteapparates bezeichnet. Auch dieses besteht aus einem mehrschichtigen Plattenepithel, welches ebenfalls nur wenige Hornschichten aufweist.

Ein wichtiges Charakteristikum von Mundschleimhaut und Zahnfleisch ist demzufolge das Fehlen bzw. die sehr schwache Ausbildung der Hornschicht. Mit dieser Hornschicht fehlt der gesamten Mundregion auch eine wichtige äußere Barriere gegenüber mechanischen, chemischen und physikalischen Einflüssen. Die Schleimhautzellen sind somit allen vorgenannten Beanspruchungen ohne Schutzschicht ausgesetzt und reagieren aus diesem Grund empfindlicher auf die durch das Zähneputzen hervorgerufenen mechanischen Beanspruchungen und die mit der Anwendung von Zahncremes verbundenen chemischen Reize.

So wertvoll Tenside für die Erhaltung der Zahngesundheit sind, können sie aus den vorgenannten Gründen auch einen nachteiligen Einfluss auf die Zellen von Zahnfleisch und Mundschleimhaut ausüben. Möglichkeiten, diese nachteiligen Wirkungen zu kompensieren, sind aus dem Stand der Technik bislang nicht bekannt.

Der vorliegenden Anmeldung lag daher die Aufgabe zugrunde, ein sowohl für die Zähne als auch für das Zahnfleisch bzw. die Mundschleimhaut optimiertes Zahnreinigungs- und Pflegemittel bereitzustellen, welches den Einsatz der für Zahnreinigung und Kariesprophylaxe unverzichtbaren Inhaltsstoffe wie Tenside ermöglicht, hierbei aber gleichzeitig deren negativen Einfluss auf das Zahnfleisch minimiert, das Zahnfleisch vitalisiert und dessen Widerstandsfähigkeit stärkt.

Die Zahnreinigung ist auch eine mechanische Beanspruchung, welche - abhängig von der Reinigungstechnik, der Bürstenkonstruktion und des während des Putzvorgangs auf den Bürstenkopf ausgeübten Drucks - dem Zahnfleisch bzw. der Mundschleimhaut kleine Verletzungen zufügen kann. Diese Mikroverletzungen werden vom Verbraucher oftmals gar nicht bemerkt, können aber im schlimmsten Fall zu Schmerzreizen oder Zahnfleischbluten während des Reinigungsvorgangs führen. Je vitaler die Mundschleimhaut bzw. das Zahnfleisch ist, desto widerstandsfähiger ist es gegenüber entsprechenden Mikroverletzungen, und desto schneller und komplikationsloser heilen diese Mikroverletzungen ab.

Es ist daher weiterhin die Aufgabe dieser Erfindung, Zahnreinigungsmittel bereitzustellen, welche das Zahnfleisch bereits während des Reinigungsvorgangs vitalisieren und seine Widerstandsfähigkeit stärken, so dass auf diese Weise die Entstehung kleiner Mikroverletzungen verhindert und - sollten doch kleine Verletzungen verursacht worden sein - die Regenerationsfähigkeit des Zahnfleisches erhöht wird.

In nicht vorhersehbarer Weise wurde nun gefunden, dass das die zuvor beschriebenen Aufgaben durch Zahnpflege- und -reinigungsmittel gelöst werden können, die neben anionischem und amphoterem Tensid weiterhin Vitamin E enthalten.

Entsprechend konzipierte Mittel reinigen die Zähne in bewährter Weise und gewährleisten eine optimale Kariesprophylaxe, üben hierbei jedoch keine nachteiligen Einflüsse auf Zahnfleisch und Mundschleimhaut aus, da sie das Zahnfleisch bzw. die Mundschleimhaut vitalisieren und deren Widerstandsfähigkeit stärken. Zudem sind Zahnfleisch und Mundschleimhaut gegenüber mechanischen Beanspruchungen, wie sie durch den Zahnreinigungsvorgang hervorgerufen werden, besser geschützt und schneller regenerationsfähig.

Unter Vitalisierung wird im Sinne der vorliegenden Erfindung eine Erhöhung der Vitalität der Zellen des Zahnfleischs und der Mundschleimhaut verstanden. Eine Erhöhung der Vitalität bedeutet eine Erhöhung der Zellüberlebensfähigkeit, welche beispielsweise nach verschiedenen Verfahren wie dem Nachweis der Zellatmungsaktivität mittels Vitalstoffen ermittelt werden kann. Die Erhöhung der Vitalität einer Zelle verbessert auch deren Widerstandsfähigkeit.

Ein erster Gegenstand der vorliegenden Erfindung ist ein Mund- und Zahnpflege- und - reinigungsmittel, enthaltend, bezogen auf sein Gesamtgewicht
a) 0,001 bis 1,5 Gew.-% Tocopherylacetat;
b) 0,05 bis 5,0 Gew.-% Natriumlaurylsulfat;
c) 0,05 bis 5,0 Gew.-% Cocamidopropylbetain
d) 0,01 bis 2,0 Gew.-% Salbeiextrakt.

Zahnpflege- und -reinigungsmittel, enthaltend
a) 0,1 Gew.-% Tocopherylacetat
b) 1,6 Gew.-% Natriumlaurylsulfat
c) 0,3 Gew.-% Cocamidopropylbetain
werden in der japanischen Patentanmeldung JP 2012-097057 A beschrieben.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Mund- und Zahnpflege- und -reinigungsmittel Tocopherylacetat. Im Zuge der zu dieser Erfindung führenden Arbeiten hat sich herausgestellt, dass sich das bzw. die erfindungsgemäßen Mittel insbesondere dann besonders vitalisierend auf Zahnfleisch und Mundschleimhaut auswirken, wenn das Tocopherylacetat in bestimmten Konzentrationsbereichen eingesetzt wird. Mund- und Zahnpflege- und - reinigungsmittel, die, bezogen auf sein Gesamtgewicht, 0,01 bis 1,0 Gew.-%, vorzugsweise 0,05 bis 0,5 Gew.-% gegebenenfalls acetyliertes Vitamin E enthalten, sind aus diesem Grund erfindungsgemäß bevorzugt.

Die Bezeichnung "gegebenenfalls acetyliertes Vitamin E" umfasst eine Reihe wirksamer Substanzen. Zu diesen Substanzen zählen insbesondere die Substanzfamilien der Tocopherole, der Tocotrienole sowie die Tocomonoenole und marinen Tocopherole (MDT, marine derived tocopherols).

Die Substanzfamilie der Tocopherole umfasst α-Tocopherol, β-Tocopherol, χ-Tocopherol, δ-Tocopherol sowie die weiteren natürlich vorkommenden Tocopherole 5,7-Dimethyltocol und 7-Methyltocol.

Alle vorgenannten Vitamin-E-Formen sind zur Lösung der erfindungsgemäßen Aufgabe geeignet. Aufgrund ihrer Wirkung besonders bevorzugt wird jedoch das α-Tocopherol. Bevorzugte Mund- und Zahnpflege- und -reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% acetyliertes α-Tocopherol enthalten.

Die Tocopherole weisen drei Stereozentren auf. Folglich existieren also von jedem der vier oben genannten Tocopherole (α-, β-, χ- und δ-Tocopherol) jeweils acht Stereoisomere. Als im Rahmen der vorliegenden Anmeldung besonders wirkungsvoll hat sich dass *RRR*-α-Tocopherol erwiesen. Besonders bevorzugte Mund- und Zahnpflege- und -reinigungsmittel sind daher dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% acetyliertes *RRR*-α-Tocopherol enthalten.

Bevorzugte Mund- und Zahnpflege- und -reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% Tocopherylacetat, vorzugsweise α-Tocopherylacetat, insbesondere *RRR*-α-Tocopherylacetat enthalten.

Als zweiten wesentlichen Inhaltsstoff enthalten die erfindungsgemäßen Mund- und Zahnpflege- und -reinigungsmittel Natriumlaurylsulfat.

Ein besonders vorteilhafter Effekt auf Zahnfleisch und Mundschleimhaut ist dann zu beobachten, wenn die Gesamtmenge an anionischen Tensiden (b), die in den erfindungsgemäßen Mitteln eingesetzt wird, auf die im Mittel enthaltende Menge an Vitamin E (a) abgestimmt ist. Erfindungsgemäß bevorzugte Mund- und Zahnpflege- und -reinigungsmittel sind dadurch gekennzeichnet, dass sie, bezogen auf ihr Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% Natriumlaurylsulfat enthalten.

Als dritten wesentlichen Inhaltsstoff enthalten die erfindungsgemäßen Mund- und Zahnpflege- und -reinigungsmittel Cocoamidopropylbetain.

Wenn die Gesamtmenge der im erfindungsgemäßen Mittel eingesetzten amphoteren Tenside (c) auf die im Mittel enthaltene Menge an gegebenenfalls acetyliertem Vitamin E (a) abgestimmt ist, so ist dies ebenfalls von besonderem Vorteil im Hinblick auf die Vitalisierung von Zahnfleisch und Mundschleimhaut. Aus diesem Grund ist es bevorzugt, wenn das Cocoamidopropylbetainbezogen auf seine Gesamtmenge im Mittel - in speziellen Mengenbereichen eingesetzt werden.

In einer weiteren besonders bevorzugten Ausführungsform ist ein erfindungsgemäßes Mund- und Zahnpflege- und -reinigungsmittel daher dadurch gekennzeichnet, dass es, bezogen auf sein Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% Cocamidopropylbetain enthält.

Die erfindungsgemäße Kombination aus anionischem und amphoterem Tensid ist schonend für das Zahnfleisch und stellt gleichzeitig eine ausreichende Reinigung der Zahnoberflächen sicher. Für die Wirkung der erfindungsgemäßen Mittel hat es sich als vorteilhaft erwiesen, die zuvor beschriebene Wirkstoffkombination aus Vitamin E, anionischem Tensid und amphoterem Tensid durch weitere spezifische Wirkstoffe zu ergänzen, wobei sich wundheilende und entzündungshemmende Stoffe und Fluoridverbindungen als besonderes wirkungsvoll erwiesen haben.

Als wundheilende und entzündungshemmende Stoffe eignen sich beispielsweise Allantoin, Azulen, Kamillenextrakten, Tocopherol, Panthenol, Bisabolol, insbesondere jedoch Salbeiextrakte. Mund- und Zahnpflege- und -reinigungsmittel nach einem der vorherigen Ansprüche, dadurch gekennzeichnet, dass sie, bezogen auf ihr Gesamtgewicht, 0,001 bis 2,0 Gew.-%, vorzugsweise 0,01 bis 1,0 Gew.-% und insbesondere 0,05 bis 0,25 Gew.-% Salbei-Blattextrakt enthalten, werden erfindungsgemäß bevorzugt.

Ein Extrakt im Sinne der vorliegenden Anmeldung ist ein Stoff oder Stoffgemisch, welches durch Extraktion gewonnen wurde. Die Angaben zum Gewichtsanteil des Extrakts am Gesamtgewicht erfindungsgemäßer Mittel beziehen sich also auf die aus dem Extraktionsgut durch Extraktion erhaltenen Stoffe oder Stoffgemische nicht jedoch auf etwaige Hilfs- oder Begleitstoffe, wie die zur Extraktion eingesetzten Lösungsmittel.

Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, daß aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.
Extrakte des Salbeis werden vor allem aus den Blättern gewonnen. Erfindungsgemäß geeignet sind sämtliche Extrakte, aus Kostengründen sind Extrakte aus den Blättern.

Die erfindungsgemäß verwendeten Granatapfelextrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Auch solventfreie Extraktionsmethoden, einschließlich der überkritischen CO₂-Extraktion sind einsetzbar.

Enthalten die erfindungsgemäßen Mund- und Zahnpflege- und -reinigungsmittel eine Fluorverbindung, so kann diese beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt.

Die Fluorverbindungen können in einer Gesamtmenge von 0,0001 bis 1,0 Gew.-%, vorzugsweise von 0,001 bis 0,35 Gew.-%, weiter bevorzugt von 0,005 bis 0,175 Gew.-% und besonders bevorzugt von 0,01 bis 0,1 Gew.-% im erfindungsgemäßen Mittel enthalten sein.

Bevorzugte erfindungsgemäße Mund- und Zahnpflege- und -reinigungsmittel sind dadurch gekennzeichnet, dass sie einen zusätzlichen Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, bevorzugt von 0,1 bis 2,5 Gew.% und insbesondere von 0,2 bis 1,1 Gew.%, jeweils bezogen auf ihr Gesamtgewicht, enthalten.

Die Zusammensetzung einiger bevorzugter Mund- und Zahnpflege- und -reinigungsmittel kann den folgenden Tabellen entnommen werden (Angaben in Gew.-% bezogen auf das Gesamtgewicht des Reinigungsmittels sofern nicht anders angegeben).

| | Formel 36 | Formel 37 | Formel 38 | Formel 39 | Formel 40 |
|---|---|---|---|---|---|
| Tocopherylacetat | 0,001 bis 1,5 | 0,01 bis 1,0 | 0,01 bis 1,0 | 0,05 bis 0,5 | 0,05 bis 0,5 |
| Natriumlaurylsulfat | 0,05 bis 5,0 | 0,1 bis 3,5 | 0,2 bis 2,5 | 0,5 bis 2,0 | 0,5 bis 2,0 |
| Cocoamidopropylbetain | 0,05 bis 5,0 | 0,1 bis 3,5 | 0,2 bis 2,5 | 0,2 bis 2,5 | 0,5 bis 2,0 |
| Salbei Extrakt | 0,001 bis 2,0 | 0,001 bis 2,0 | 0,01 bis 1,0 | 0,01 bis 1,0 | 0,05 bis 0,25 |
| Misc | add 100 | add 100 | add 100 | add 100 | add 100 |

| | Formel 76 | Formel 77 | Formel 78 | Formel 79 | Formel 80 |
|---|---|---|---|---|---|
| Tocopherylacetat | 0,001 bis 1,5 | 0,01 bis 1,0 | 0,01 bis 1,0 | 0,05 bis 0,5 | 0,05 bis 0,5 |
| Natriumlaurylsulfat | 0,05 bis 5,0 | 0,1 bis 3,5 | 0,2 bis 2,5 | 0,5 bis 2,0 | 0,5 bis 2,0 |
| Cocoamidopropylbetain | 0,05 bis 5,0 | 0,1 bis 3,5 | 0,2 bis 2,5 | 0,2 bis 2,5 | 0,5 bis 2,0 |
| Salbei Extrakt | 0,001 bis 2,0 | 0,001 bis 2,0 | 0,01 bis 1,0 | 0,01 bis 1,0 | 0,05 bis 0,25 |
| Fluoridverbindung | 0,01 bis 5,0 | 0,1 bis 2,5 | 0,1 bis 2,5 | 0,2 bis 1,1 | 0,2 bis 1,1 |
| Misc | add 100 | add 100 | add 100 | add 100 | add 100 |

Zusätzlich zu den erfindungswesentlichen Komponenten (a), (b), (c) und (d) enthalten die erfindungsgemäßen Mittel vorzugsweise weitere pflege- und reinigungsaktive Inhaltsstoffe.

Die erfindungsgemäßen Zusammensetzungen können zusätzlich mindestens ein Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, dass sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders geeignet sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, besonders geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident® 8 (Evonik) und Sorbosil® AC 39 (PQ Corporation). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pas aufweisen.

Zahnpasten, die eine deutlich höhere Viskosität von mehr als 100 Pas (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 - 250 m²/g zu, z.B. das Handelsprodukt Sident® 22S.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben sich als besonders effizient bei der Lösung der erfindungsgemäßen Aufgabe erwiesen.

Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und -reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g.

Besonders bevorzugte erfindungsgemäße Mund und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

Mund- und Zahnpflege- und reinigungsmittel, insbesondere Zahnpasten, können zusätzlich auch Substanzen enthalten, die gegen Plaque und/oder Zahnstein wirksam sind. Hierzu eignen sich beispielsweise antimikrobielle Stoffe und Konsvervierungsmittel (Plaque) oder Chelatbildner (Zahnstein).

Erfindungsgemäß bevorzugte Mund- und Zahnpflege- und -reinigungsmittel sind dadurch gekennzeichnet, dass sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O7, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Träger für die Zahnpasten, der die Einstellung einer geeigneten Konsistenz für die Dosierung aus Tuben, Spendebehältern oder flexiblen Flaschen ermöglicht, eignet sich beispielsweise eine Kombination aus Feuchthaltemitteln, Bindemitteln und Wasser.

Geeignete Feuchthaltemittel sind beispielsweise Sorbit, Glycerin oder 1,2-Propylenglycol. Neben diesen Stoffen, von denen mindestens einer in Mengen von mindestens 10 Gew.-% in den erfindungsgemäßen Mitteln enthalten ist, eignen sich als Feuchthaltemittel vorzugsweise auch Alkohole mit mindestens 2 OH-Gruppen, vorzugsweise Mannit, Xylitol, Polyethylenglycol, Polypropylenglycol und deren Mischungen. Ein bevorzugtes Mund- und Zahnpflege- und - reinigungsmittel ist dadurch gekennzeichnet, dass es weiterhin mindestens einen mehrwertigen Alkohol aus der Gruppe Sorbit und/oder Glycerin und/oder 1,2-Propylenglycol enthält.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.
Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.

Die erfindungsgemäßen Mittel können in einem kosmetischen, nicht therapeutischen Verfahren zur Reinigung der Zähne bei gleichzeitiger Vitalisierung bzw. Kräftigung des Zahnfleisches und der Mundschleimhaut eingesetzt werden

Ein weiterer Gegenstand der vorliegenden Erfindung ist ein erfindungsgemäßes Mittel zur Anwendung in einem Verfahren zur Reinigung der Zähne bei gleichzeitiger Vitalisierung des Zahnfleisches und der Mundschleimhaut, dadurch gekennzeichnet, dass
i) 0,5 bis 5,0 g eines Mittels nach einem der Ansprüche 1 bis 8 auf eine Zahnbürste aufgetragen werden,
ii) das Mittel anschließend mit Hilfe der Zahnbürste auf den Zähnen und/oder dem die Zähne umgebenden Zahnfleisch und/oder der Mundschleimhaut appliziert wird,
iii) das Mittel dort für einen Zeitraum von 30 bis 300 Sekunden belassen wird, während gleichzeitig die Zähne mit der Zahnbürste gereinigt werden und
iv) das Mittel anschließend mit Wasser wieder von Zähnen, Zahnfleisch und Mundschleimhaut abgespült wird.

### Beispiele:

### 1. Messung der Zellvitalität

Als Maß für die Vitalität kann die metabolische Aktivität von Gingiva Fibroblasten nach Inkubation mit Tocopherylacetat gemessen werden. Das chromogene Tetrazoliumsalz WST-1 wird durch stoffwechselaktive Succinatdehydrogenasen lebender Zellen reduktiv aufgespalten, wobei ein wasserlösliches Formazansalz entsteht. Durch UV/VIS-spektroskopische Vermessung der Absorptionsintensität kann die Konzentration der intermediär gebildeten Formazansalze bestimmt werden, welche in Korrelation zur Aktivität der Succinatdehydrogenase steht.

Der WST-Vitalitätstest wurde in einer 96-Well Mikrotiterplatte in 6-fach Bestimmung durchgeführt. Dafür wurde pro Well eine definierte Zellzahl von Gingiva Fibroplasten in einem Volumen von 50 µl (microliter) ausgesät. Anschließend wurde Tocopherylacetat in absteigenden Konzentrationen dazugegeben. Nach 24 Stunden Inkubation wurden 10 µl des in Medium verdünnten Tetrazolium-Salzes WST-1 zu jedem Ansatz pipettiert. Es folgte eine dreistündige Inkubation bei 37°C und 5 % Kohlenstoffdioxid, bevor die Absorption bei einer Wellenlänge von 450 nm (Referenzwellenlänge 650 nm) in einem Spektrometer (ELISA-Reader) gemessen wurde. Die Werte der unbehandelten Kontrolle wurden gleich 100 % gesetzt und die Werte der behandelten Proben darauf bezogen.

Für jede Tocopherylacetat-Konzentration wurde eine 6-fach Bestimmung durchgeführt, aus den 6 Messwerten wurde jeweils der Mittelwert gebildet.

**Tabelle 1: Einfluss von Tocopherylacetat auf die Zellaktivität**

| | Konz. [Gew.-%] | Vitalität [%] Mittelwert | StandardAbweichung |
|---|---|---|---|
| unbehandelt | --- | 100,0 | 9,9 |
| | 0,01 | 150,5 | 7,3 |
| | 0,005 | 137,7 | 25,5 |
| Tocopherylacetat | 0,001 | 141,4 | 11,7 |
| | 0,0005 | 132,9 | 16,0 |

Die Ergebnisse zeigen, dass die Applikation von Tocopherylacetat in geringen Konzentrationen in Gingiva-Zellen zu einer signifikanten Steigerung der Vitalität führte.

### 2.Messung des Zellenergieinhalts

ATP (Adenosintriphosphat) ist die universelle Speicherform für chemische Energie in Zellen. Die ATP-Bestimmung erfolgt mit Hilfe des ATPLiteTM-M Assays (Packard). Das Testprinzip dieses Assays beruht darauf, dass die Luciferase von Photinus pyralis eine Reaktion katalysiert, bei der in Gegenwart von ATP D-Luciferin in Oxyluciferin umgewandelt wird. Bei dieser Reaktion wird grünes Licht emittiert, das mit einem Luminometer gemessen werden kann. Das emittierte Biolumineszenzlicht ist proportional zur Menge des vorhandenen ATP. Zur Bestimmung der ATP-Aktivität in Gingiva Fibroblasten werden diese in geeigneter Weise unter Erhalt ihrer spezifischen Eigenschaften vorkultiviert (DE10162814) und in eine 96well-Zellkulturschale überführt. Die Behandlung mit dem Substanzgemisch erfolgte über 24 Stunden gegen eine unbehandelte Kontrolle. Anschließend wurden die Zellen mit jeweils 50 µl/Kavität eines im Testkit enthaltenen Lysepuffer für 5 min auf einem Schüttler lysiert. Danach wurden die Zellen für weitere 5 min mit jeweils 50 µl/Kavität mit der mitgelieferten Substratlösung auf dem Schüttler inkubiert und anschließend das Reaktionsgemisch in eine schwarze Mikrotiterplatte überführt. Nach einer Inkubationszeit von 10 min in der Dunkelheit erfolgte die Messung.

**Tabelle 2: Einfluss von Tocopherylacetat auf den Zellenergieinhalt**

| | Konz. [Gew.-%] | ATP-Gehalt [%] Mittelwert | StandardAbweichung |
|---|---|---|---|
| unbehandelt | --- | 100,0 | 3,7 |
| | 0,01 | 128,5 | 17,7 |
| Tocopherylacetat | 0,005 | 138,9 | 14,8 |
| | 0,0025 | 125,4 | 5,9 |

Die Ergebnisse zeigen, dass die Applikation von Tocopherylacetat in geringen Konzentrationen in Gingiva-Zellen zu einer signifikanten Steigerung des ATP-Gehaltes führt.

### 3. Messung der Schleimhautverträglichkeit

Die tensidischen Zehncremeformulierungen wurden im HET-CAM-Test untersucht. Der Test stellt ein in vitro Prüfverfahren dar, das zur Beurteilung der Schleimhautverträglichkeit von Stoffen und Formulierungen - insbesondere tensidischen Verbindungen - geeignet ist.

Die Prüfmuster wurden im HET-CAM-Test mittels Reaktionszeitmethode (Reaktionsverlauf über 30 Sekunden) unverdünnt untersucht. In diesem Test haben die nachfolgend unter Punkt 4. angeführten Zahncremeformulierungen ein geringes Reizpotential aufgewiesen.

### 4. Formulierungen

Es wurden die folgenden nicht erfindungsgemäßen Zahncremeformulierungen hergestellt:

| | Bsp. 1 | Bsp. 2 | Bsp. 3 |
|---|---|---|---|
| Rostoff | | | |
| | Menge [Gew.-%] | Menge [Gew.-%] | Menge [Gew.-%] |
| Sorbitol | 45 | 60 | 50 |
| Poliermittel | 10 | 10 | 10 |
| Saccharin, Natriumsalz | 0,5 | 0,5 | 0,5 |
| Natriumlaurylsulfat | 1,0 | 1,0 | 0,9 |
| Cocoamidopropylbetain | 0,5 | 0,6 | 0,8 |
| Tocopherylacetat | 0,04 | 0,06 | 0,1 |
| Natriumfluorid | 0,1 | 0,2 | 0,15 |
| Aroma | 1,0 | 1,0 | 1,0 |
| Ethanol | --- | 1,5 | --- |
| Xanthan Gum | 0,5 | 0,5 | 0,5 |
| Maleicsäureanhydrid/Methylvinylether Copolymer | 1,0 | 1,0 | 1,0 |
| Wasser | ad 100 | ad 100 | ad 100 |

Dialkali- oder Tetraalkalimetallpyrophosphat- Salze, z. B. Na₄P₂O₇, K₄P ₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Träger für die Zahnpasten, der die Einstellung einer geeigneten Konsistenz für die Dosierung aus Tuben, Spendebehältern oder flexiblen Flaschen ermöglicht, eignet sich beispielsweise eine Kombination aus Feuchthaltemitteln, Bindemitteln und Wasser.

Geeignete Feuchthaltemittel sind beispielsweise Sorbit, Glycerin oder 1,2-Propylenglycol. Neben diesen Stoffen, von denen mindestens einer in Mengen von mindestens 10 Gew.-% in den erfindungsgemäßen Mitteln enthalten ist, eignen sich als Feuchthaltemittel vorzugsweise auch Alkohole mit mindestens 2 OH-Gruppen, vorzugsweise Mannit, Xylitol, Polyethylenglycol, Polypropylenglycol und deren Mischungen. Ein bevorzugtes Mund- und Zahnpflege- und - reinigungsmittel ist dadurch gekennzeichnet, dass es weiterhin mindestens einen mehrwertigen Alkohol aus der Gruppe Sorbit und/oder Glycerin und/oder 1,2-Propylenglycol enthält.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.

Die erfindungsgemäßen Mittel können in einem kosmetischen, nicht therapeutischen Verfahren zur Reinigung der Zähne bei gleichzeitiger Vitalisierung bzw. Kräftigung des Zahnfleisches und der Mundschleimhaut eingesetzt werden Wir beschreiben auch ein kosmetisches, nicht therapeutisches Verfahren zur Reinigung der Zähne bei gleichzeitiger Vitalisierung des Zahnfleisches und der Mundschleimhaut, dadurch gekennzeichnet, dass
i) 0,5 bis 5,0 g eines Mittels nach einem der Ansprüche 1 bis 8 auf eine Zahnbürste aufgetragen werden,
ii) das Mittel anschließend mit Hilfe der Zahnbürste auf den Zähnen und/oder dem die Zähne umgebenden Zahnfleisch und/oder der Mundschleimhaut appliziert wird,
iii) das Mittel dort für einen Zeitraum von 30 bis 300 Sekunden belassen wird, während gleichzeitig die Zähne mit der Zahnbürste gereinigt werden und
iv) das Mittel anschließend mit Wasser wieder von Zähnen, Zahnfleisch und Mundschleimhaut abgespült wird.

Im Rahmen der vorliegenden Erfindung wurde überraschend festgestellt, dass gegebenenfalls acetyliertes Vitamin E eine herausragende Eignung zur Vitalisierung der Zellen von Zahnfleisch und Mundschleimhaut besitzt. Ein weiterer Gegenstand der vorliegenden Erfindung ist daher die kosmetische, nicht therapeutische Verwendung von gegebenenfalls acetyliertem Vitamin E
- zur Stimulation gingivaler Zellen, vorzugsweise gingivaler Fibroblasten
- zur Vitalisierung und Kräftigung des Zahnfleischs und der Mundschleimhaut und/oder
- zur Erhöhung der Zellüberlebensfähigkeit und/oder
- zur Verbesserung der Zahnfleischregeneration und/oder
- zur Verjüngung des Zahnfleisches und/oder
- zum Schutz vor Zahnfleischentzündungen.

Bezüglich bevorzugter Ausführungsformen des erfindungsgemäßen Verfahrens und der erfindungsgemäßen Verwendung gilt mutatis mutandis das zu den erfindungsgemäßen Mitteln Gesagte.

### Beispiele:

### 1. Messung der Zellvitalität

Als Maß für die Vitalität kann die metabolische Aktivität von Gingiva Fibroblasten nach Inkubation mit Tocopherylacetat gemessen werden. Das chromogene Tetrazoliumsalz WST-1 wird durch stoffwechselaktive Succinatdehydrogenasen lebender Zellen reduktiv aufgespalten, wobei ein wasserlösliches Formazansalz entsteht. Durch UV/VIS-spektroskopische Vermessung der Absorptionsintensität kann die Konzentration der intermediär gebildeten Formazansalze bestimmt werden, welche in Korrelation zur Aktivität der Succinatdehydrogenase steht.

Der WST-Vitalitätstest wurde in einer 96-Well Mikrotiterplatte in 6-fach Bestimmung durchgeführt. Dafür wurde pro Well eine definierte Zellzahl von Gingiva Fibroplasten in einem Volumen von 50 µl (microliter) ausgesät. Anschließend wurde Tocopherylacetat in absteigenden Konzentrationen dazugegeben. Nach 24 Stunden Inkubation wurden 10 µl des in Medium verdünnten Tetrazolium-Salzes WST-1 zu jedem Ansatz pipettiert. Es folgte eine dreistündige Inkubation bei 37 °C und 5 % Kohlenstoffdioxid, bevor die Absorption bei einer Wellenlänge von 450 nm (Referenzwellenlänge 650 nm) in einem Spektrometer (ELISA-Reader) gemessen wurde. Die Werte der unbehandelten Kontrolle wurden gleich 100 % gesetzt und die Werte der behandelten Proben darauf bezogen.

Für jede Tocopherylacetat-Konzentration wurde eine 6-fach Bestimmung durchgeführt, aus den 6 Messwerten wurde jeweils der Mittelwert gebildet.

**Tabelle 1: Einfluss von Tocopherylacetat auf die Zellaktivität**

| | Konz. [Gew.-%] | Vitalität [%] Mittelwert | StandardAbweichung |
|---|---|---|---|
| unbehandelt | --- | 100,0 | 9,9 |
| | 0,01 | 150,5 | 7,3 |
| | 0,005 | 137,7 | 25,5 |
| Tocopherylacetat | 0,001 | 141,4 | 11,7 |
| | 0,0005 | 132,9 | 16,0 |

Die Ergebnisse zeigen, dass die Applikation von Tocopherylacetat in geringen Konzentrationen in Gingiva-Zellen zu einer signifikanten Steigerung der Vitalität führte.

### 2.Messung des Zellenergieinhalts

ATP (Adenosintriphosphat) ist die universelle Speicherform für chemische Energie in Zellen. Die ATP-Bestimmung erfolgt mit Hilfe des ATPLiteTM-M Assays (Packard). Das Testprinzip dieses Assays beruht darauf, dass die Luciferase von Photinus pyralis eine Reaktion katalysiert, bei der in Gegenwart von ATP D-Luciferin in Oxyluciferin umgewandelt wird. Bei dieser Reaktion wird grünes Licht emittiert, das mit einem Luminometer gemessen werden kann. Das emittierte Biolumineszenzlicht ist proportional zur Menge des vorhandenen ATP. Zur Bestimmung der ATP-Aktivität in Gingiva Fibroblasten werden diese in geeigneter Weise unter Erhalt ihrer spezifischen Eigenschaften vorkultiviert (DE10162814) und in eine 96well-Zellkulturschale überführt. Die Behandlung mit dem Substanzgemisch erfolgte über 24 Stunden gegen eine unbehandelte Kontrolle. Anschließend wurden die Zellen mit jeweils 50 µl/Kavität eines im Testkit enthaltenen Lysepuffer für 5 min auf einem Schüttler lysiert. Danach wurden die Zellen für weitere 5 min mit jeweils 50 µl/Kavität mit der mitgelieferten Substratlösung auf dem Schüttler inkubiert und anschließend das Reaktionsgemisch in eine schwarze Mikrotiterplatte überführt. Nach einer Inkubationszeit von 10 min in der Dunkelheit erfolgte die Messung.

**Tabelle 2: Einfluss von Tocopherylacetat auf den Zellenergieinhalt**

| | Konz. [Gew.-%] | ATP-Gehalt [%] Mittelwert | StandardAbweichung |
|---|---|---|---|
| unbehandelt | --- | 100,0 | 3,7 |
| | 0,01 | 128,5 | 17,7 |
| Tocopherylacetat | 0,005 | 138,9 | 14,8 |
| | 0,0025 | 125,4 | 5,9 |

Die Ergebnisse zeigen, dass die Applikation von Tocopherylacetat in geringen Konzentrationen in Gingiva-Zellen zu einer signifikanten Steigerung des ATP-Gehaltes führt.

### 3. Messung der Schleimhautverträglichkeit

Die tensidischen Zehncremeformulierungen wurden im HET-CAM-Test untersucht. Der Test stellt ein in vitro Prüfverfahren dar, das zur Beurteilung der Schleimhautverträglichkeit von Stoffen und Formulierungen - insbesondere tensidischen Verbindungen - geeignet ist.

Die Prüfmuster wurden im HET-CAM-Test mittels Reaktionszeitmethode (Reaktionsverlauf über 30 Sekunden) unverdünnt untersucht. In diesem Test haben die nachfolgend unter Punkt 4. angeführten Zahncremeformulierungen ein geringes Reizpotential aufgewiesen.

### 4. Formulierungen

Es wurden die folgenden Zahncremeformulierungen hergestellt:

| | Bsp.1 | Bsp.2 | Bsp. 3 |
|---|---|---|---|
| Rostoff | Menge [Gew.-%] | Menge [Gew.-%] | Menge [Gew.-%] |
| Sorbitol | 45 | 60 | 50 |
| Poliermittel | 10 | 10 | 10 |
| Saccharin, Natriumsalz | 0,5 | 0,5 | 0,5 |
| Natriumlaurylsulfat | 1,0 | 1,0 | 0,9 |
| Cocoamidopropylbetain | 0,5 | 0,6 | 0,8 |
| Tocopherylacetat | 0,04 | 0,06 | 0,1 |
| Natriumfluorid | 0,1 | 0,2 | 0,15 |
| Aroma | 1,0 | 1,0 | 1,0 |
| Ethanol | --- | 1,5 | --- |
| Xanthan Gum | 0,5 | 0,5 | 0,5 |
| Maleicsäureanhydrid/Methylvinylether Copolymer | 1,0 | 1,0 | 1,0 |
| Wasser | ad 100 | ad 100 | ad 100 |

## Patentansprüche

1. Mund- und Zahnpflege- und -reinigungsmittel, enthaltend, bezogen auf sein Gesamtgewicht
a) 0,001 bis 1,5 Gew.-% Tocopherylacetat;
b) 0,05 bis 5,0 Gew.-%, Natriumlaurylsulfat;
c) 0,05 bis 5,0 Gew.-%, Cocamidopropylbetain
d) 0,01 bis 2,0 Gew.-% Salbeiblattextrakt

2. Mund- und Zahnpflege- und -reinigungsmittel nach einem der vorherigen Ansprüche, **dadurch gekennzeichnet, dass** es, bezogen auf sein Gesamtgewicht, vorzugsweise 0,01 bis 1,0 Gew.-%, und insbesondere 0,05 bis 0,5 Gew.-% Tocopherylacetat enthält.

3. Mund- und Zahnpflege- und -reinigungsmittel nach einem der vorherigen Ansprüche, **dadurch gekennzeichnet, dass** es, bezogen auf sein Gesamtgewicht, vorzugsweise 0,1 bis 3,5 Gew.-%, besonders bevorzugt 0,2 bis 2,5 Gew.-% und insbesondere 0,5 bis 2,0 Gew.-% Natriumlaurylsulfat enthält.

4. Mund- und Zahnpflege- und -reinigungsmittel nach einem der vorherigen Ansprüche, **dadurch gekennzeichnet, dass** es, bezogen auf sein Gesamtgewicht, vorzugsweise 0,1 bis 3,5 Gew.-%, besonders bevorzugt 0,2 bis 2,5 Gew.-% und insbesondere 0,5 bis 2,0 Gew.-% Cocamidopropylbetain enthält.

5. Mund- und Zahnpflege- und -reinigungsmittel nach einem der vorherigen Ansprüche, **dadurch gekennzeichnet, dass** es, bezogen auf sein Gesamtgewicht, 0,01 bis 1,0 Gew.-% und insbesondere 0,05 bis 0,25 Gew.-% Salbei-Blattextrakt enthält.

6. Mund- und Zahnpflege- und -reinigungsmittel nach einem der vorherigen Ansprüche, **dadurch gekennzeichnet, dass** es, bezogen auf sein Gesamtgewicht, einen zusätzlichen Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, bevorzugt von 0,1 bis 2,5 Gew.% und insbesondere von 0,2 bis 1,1 Gew.% enthält.

7. Mund- und Zahnpflege- und -reinigungsmittel nach einem der vorherigen Ansprüche, **dadurch gekennzeichnet, dass** es weiterhin mindestens einen mehrwertigen Alkohol aus der Gruppe Sorbit und/oder Glycerin und/oder 1,2-Propylenglycol enthält.

8. Mittel nach einem der Ansprüche 1 bis 7 zur Anwendung in einem Verfahren zur Reinigung der Zähne bei gleichzeitiger Vitalisierung des Zahnfleisches und der Mundschleimhaut, **dadurch gekennzeichnet, dass**
i) 0,5 bis 5,0 g eines Mittels nach einem der Ansprüche 1 bis 7 auf eine Zahnbürste aufgetragen werden,
ii) das Mittel anschließend mit Hilfe der Zahnbürste auf den Zähnen und/oder dem die Zähne umgebenden Zahnfleisch und/oder der Mundschleimhaut appliziert wird,
iii) das Mittel dort für einen Zeitraum von 30 bis 300 Sekunden belassen wird, während gleichzeitig die Zähne mit der Zahnbürste gereinigt werden und
iv) das Mittel anschließend mit Wasser wieder von Zähnen, Zahnfleisch und Mundschleimhaut abgespült wird.

## Claims

1. An oral and dental care and cleaning agent, containing, based on the total weight thereof,
a) from 0.001 to 1.5 wt.% tocopheryl acetate;
b) from 0.05 to 5.0 wt.% sodium lauryl sulfate;
c) from 0.05 to 5.0 wt.% cocamidopropyl betaine;
d) from 0.01 to 2.0 wt.% sage leaf extract.

2. The oral and dental care and cleaning agent according to the preceding claim, **characterized in that** it contains, based on the total weight thereof, preferably from 0.01 to 1.0 wt.% and in particular from 0.05 to 0.5 wt.% tocopheryl acetate.

3. The oral and dental care and cleaning agent according to one of the preceding claims, **characterized in that** it contains, based on the total weight thereof, preferably from 0.1 to 3.5 wt.%, particularly preferably from 0.2 to 2.5 wt.% and in particular from 0.5 to 2.0 wt.% sodium lauryl sulfate.

4. The oral and dental care and cleaning agent according to one of the preceding claims, **characterized in that** it contains, based on the total weight thereof, preferably from 0.1 to 3.5 wt.%, particularly preferably from 0.2 to 2.5 wt.% and in particular from 0.5 to 2.0 wt.% cocamidopropyl betaine.

5. The oral and dental care and cleaning agent according to one of the preceding claims, **characterized in that** it contains, based on the total weight thereof, from 0.01 to 1.0 wt.% and in particular from 0.05 to 0.25 wt.% sage leaf extract.

6. The oral and dental care and cleaning agent according to one of the preceding claims, **characterized in that** it contains, based on the total weight thereof, an additional anticariogenic active ingredient, preferably fluorine compound(s), in particular sodium fluoride, potassium fluoride, sodium monofluorophosphate, zinc fluoride, stannous fluoride and sodium fluorosilicate, preferably in amounts of from 0.01 to 5 wt.%, preferably from 0.1 to 2.5 wt.% and in particular from 0.2 to 1.1 wt.%.

7. The oral and dental care and cleaning agent according to one of the preceding claims, **characterized in that** it further contains at least one polyhydric alcohol from the group comprising sorbitol and/or glycerol and/or 1,2-propylene glycol.

8. The agent according to one of claims 1 to 7 for use in a method for cleaning teeth while also revitalizing the gums and oral mucosa, **characterized in that**
i) from 0.5 to 5.0 g of an agent according to one of claims 1 to 7 is applied to a toothbrush,
ii) the agent is then applied to the teeth and/or the gums surrounding the teeth and/or the oral mucosa by means of the toothbrush,
iii) the agent is left there for a period of 30 to 300 seconds while the teeth are being cleaned using the toothbrush, and
iv) the agent is then rinsed off the teeth, gums and oral mucosa using water.

## Revendications

1. Agent de nettoyage et de soins buccodentaire contenant, sur la base de son poids total,
a) de 0,001 à 1,5% en poids d'acétate de tocophéryle ;
b) de 0,05 à 5,0% en poids de laurylsulfate de sodium ;
c) de 0,05 à 5,0% en poids de cocamidopropylbétaïne ;
d) de 0,01 à 2,0% en poids d'extrait de feuille de sauge.

2. Agent de nettoyage et de soins buccodentaire selon l'une des revendications précédentes, **caractérisé en ce qu'**il contient, sur la base de son poids total, de préférence de 0,01 à 1,0% en poids, et en particulier de 0,05 à 0,5% d'acétate de tocophéryle.

3. Agent de nettoyage et de soins buccodentaire selon l'une des revendications précédentes, **caractérisé en ce qu'**il contient, sur la base de son poids total, de préférence de 0,1 à 3,5% en poids, de manière particulièrement préférée de 0,2 à 2,5% en poids, et en particulier de 0,5 à 2,0% en poids de laurylsulfate de sodium.

4. Agent de nettoyage et de soins buccodentaire selon l'une des revendications précédentes, **caractérisé en ce qu'**il contient, sur la base de son poids total, de préférence 0,1 à 3,5% en poids, de manière particulièrement préférée de 0,2 à 2,5% et en particulier de 0,5 à 2,0% en poids de cocamidopropylbétaïne.

5. Agent de nettoyage et de soins buccodentaire selon l'une des revendications précédentes, **caractérisé en ce qu'**il contient, sur la base de son poids total, de 0,01 à 1,0% en poids et en particulier de 0,05 à 0,25% en poids d'extrait de feuille de sauge.

6. Agent de nettoyage et de soins buccodentaire selon l'une des revendications précédentes, **caractérisé en ce qu'**il contient, sur la base de son poids total, un agent anti-caries supplémentaire, de préférence un ou plusieurs composés fluorés, en particulier du fluorure de sodium, du fluorure de potassium, du monofluorophosphate de sodium, du fluorure de zinc, du fluorure stanneux et du fluorosilicate de sodium, de préférence dans des quantités de 0,01 à 5% en poids, de préférence de 0,1 à 2,5.% en poids, et en particulier de 0,2 à 1,1% en poids.

7. Agent de nettoyage et de soins buccodentaire selon l'une des revendications précédentes, **caractérisé en ce qu'**il contient en outre au moins un alcool polyvalent du groupe comportant le sorbitol et/ou le glycérol et/ou le 1,2-propylène-glycol.

8. Agent selon l'une des revendications 1 à 7 destiné à être utilisé dans un procédé de nettoyage des dents et de revitalisation simultanée des gencives et de la muqueuse buccale, **caractérisé en ce que**
i) 0,5 à 5,0 g d'un agent selon l'une des revendications 1 à 7 sont appliqués sur une brosse à dents,
ii) l'agent est ensuite appliqué à l'aide de la brosse à dents sur les dents et/ou la gencive entourant les dents et/ou la muqueuse buccale,
iii) l'agent est laissé pendant une période de 30 à 300 secondes tandis que les dents sont nettoyés avec la brosse à dents et
iv) l'agent est ensuite rincé à nouveau des dents, des gencives et de la muqueuse buccale avec de l'eau.
